# DEMANDE DE BREVET EUROPEEN

(11) **EP 2 510 808 A1**
(43) Date de publication de la demande: **17.10.2012**
(21) Numéro de dépôt: 12163597.3
(22) Date de dépôt: 10.04.2012
(51) Int. Cl.: A23K 1/16, A23K 1/18, A61K 36/00

(54) **Utilisation de tanins hydrolysables dans l'alimentation animale**

(30) Priorité: 11.04.2011 FR 1153143
(71) Demandeur: T L, 62000 Arras (FR)
(72) Inventeur: Tierny, Jean-Benoit, 62000 ARRAS (FR)
(74) Mandataire: Cabinet Plasseraud

(57) **Abrégé**

L'invention concerne l'utilisation d'au moins un ellagitanin pour réduire l'absorption du fer ingéré, notamment contenu dans un aliment par un animal, notamment un mammifère ainsi qu'un aliment pour animaux et un complément alimentaire pour animaux comprenant un tel tanin.

## Description

L'invention concerne l'utilisation d'au moins un tanin hydrolysable pour réduire l'absorption par un animal du fer ingéré, notamment du fer contenu dans un aliment. L'invention concerne également un aliment pour animaux ainsi qu'un complément alimentaire pour animaux comprenant au moins un tel tanin hydrolysable.

Dans certains pays, notamment en France, en Belgique et aux Pays Bas, les habitudes de consommation de viande de veau sont orientées vers une viande de couleur claire. L'obtention de ce critère est déterminante dans le cadre de la filière veau de boucherie.

La pigmention (coloration) rouge de la viande provient en partie du fait que le fer contenu dans les aliments est absorbé au niveau de l'appareil digestif de l'animal et se fixe sur ses globules rouges. Plus la quantité de fer absorbée est élevée, plus la viande est rouge.

Pendant la période d'élevage, les veaux de boucherie reçoivent une alimentation appropriée à leur âge, à leur poids et à leurs besoins comportementaux et physiologiques pour assurer leur santé, favoriser leur bien-être et pour leur permettre d'avoir un comportement normal et un développement normal du rumen permis par la distribution d'aliments fibreux.

Or la teneur en fer des aliments fibreux est significativement supérieure à celle des aliments d'allaitement. Le panel de matières premières disponibles pour répondre au bien-être des animaux et garantir la maîtrise de la couleur claire de la viande est extrêmement limité. L'utilisation de matières premières non répertoriées classiquement dans l'alimentation des veaux de boucherie pour leur teneur en fer, va entraîner l'assimilation de quantités de fer plus importantes et une perte de maîtrise de la qualité « couleur » de la viande avec des impacts économiques importants.

Suite à de nombreux essais, un lien a été démontré entre l'utilisation d'aliments riches en fer et l'incorporation d'un tanin hydrolysable. Ce dernier permet la maîtrise du niveau individuel et moyen de la couleur d'un lot d'animaux tout en respectant les seuils réglementaires.

A la connaissance des inventeurs, il n'existe aucun produit sur le marché qui permette de maîtriser l'absorption du fer par les animaux au niveau de l'appareil digestif et ainsi de maîtriser l'hématocrite sans diminution significative des performances zootechniques (performances de croissance).

Un but de l'invention est donc de proposer un moyen pour maîtriser les assimilations de fer contenu dans les aliments par les animaux, notamment des veaux de boucherie, au niveau de l'appareil digestif.

D'autres buts et avantages apparaîtront à la lecture de la description non limitative qui va suivre.

Il est du mérite des inventeurs d'avoir trouvé, après de longs et intenses travaux de recherche, que ce but pouvait être atteint en faisant ingérer aux animaux, notamment aux veaux, un tanin hydrolysable, en particulier en l'ajoutant dans l'alimentation de ceux-ci.

Un objet de l'invention est donc l'utilisation non-thérapeutique d'au moins un tanin hydrolysable pour réduire l'absorption par un animal, notamment un mammifère et plus particulièrement un veau, du fer ingéré, notamment contenu dans un aliment.

Au sens de la présente invention, on entend par l'expression « maîtriser l'absorption du fer contenu dans un aliment par un animal » la maîtrise du taux de fer dans le sang à un niveau souhaité tout en respectant le bien-être animal et la réglementation en vigueur. C'est-à-dire l'hématocrite de l'animal recevant au moins un tanin hydrolysable au sens de l'invention est inférieur à l'hématocrite d'un animal alimenté par le même aliment.

Pour un veau de boucherie, on cible actuellement un hématocrite d'environ 23 à 24% au moment de l'abattage (154 à 161 jours d'élevage). A ce niveau d'hématocrite, la viande sera de couleur claire (blanche et rosée) telle que désirée par les consommateurs.

Les tanins hydrolysables sont des esters du glucose (ou de composés apparentés) et de molécules d'acide-phénol : acide gallique (tanins galliques) ou acide hexahydrodiphénique (HHDP) et ses dérivés (ellagitanins ou tanins éllagiques).

Au sens de la présente invention, le terme tanin hydrolysable est utilisé pour signifier un ellagitanin. Les ellagitanins sont formés autour d'un sucre (glucose ou polyol dérivé du D-glucose) comportant plusieurs liaisons esters d'acide hexahydroxydiphénique (HHDP) (ou de ses dérivés DHHDP, acide chébulique). Ils sont produits à partir des gallotanins par couplage oxydatif C-2-C-2' d'au moins deux unités galloyles.

Sans vouloir être liés par une quelconque théorie, les inventeurs pensent que les ellagitanins chélatent le fer alimentaire et que le fer ainsi chélaté ne peut pas être absorbé au niveau de l'appareil digestif. Ceci résulte en un hématocrite inférieur à la normale des animaux recevant un tanin hydrolysable (ellagitanin) tel que défini dans la présente demande.

En complétant un aliment par une quantité adaptée d'au moins un tanin hydrolysable (ellagitanin), il est possible de réguler le taux d'absorption du fer alimentaire au niveau de l'appareil digestif afin d'atteindre un niveau d'hématocrite prédéfini sans diminution significative des performances zootechniques. Le tanin hydrolysable peut être incorporé dans la ration alimentaire de l'animal ou donné séparément.

Du fait que les tanins hydrolysables (ellagitanins) permettent de réduire l'absorption du fer alimentaire au niveau de l'appareil digestif, il est donc possible d'utiliser des aliments naturellement plus riches en fer, tout en gardant le taux d'absorption de fer constant par rapport à un aliment de référence moins riche en fer. Dans le cadre de l'élevage de veaux de boucherie, ceci permet d'utiliser des aliments plus riches en fer que ceux actuellement utilisés, tout en gardant essentiellement les mêmes performances zootechniques. Ceci présente un avantage économique non négligeable du fait que les aliments plus riches en fer sont généralement moins chers que les aliments pauvres en fer actuellement utilisés dans l'élevage des veaux de boucherie.

On trouve les ellagitanins dans une multitude de végétaux. A titre d'exemples non limitatifs de végétaux contenant des ellagitanins, on peut citer le châtaignier, le chêne et le roncier.

Le bois de châtaignier contient une teneur importante (environ 5%) d'ellagitanins. De ce fait, les ellagitanins extraits du bois de châtaignier sont particulièrement intéressants pour la présente invention.

Dans un mode de réalisation, on utilise donc un extrait végétal comprenant au moins un ellagitanin.

De préférence, l'extrait végétal est donc choisi parmi les extraits de bois de châtaignier, bois de chêne ou de roncier. Plus préférentiellement, l'extrait végétal est un extrait de bois de châtaignier ou un extrait de bois de chêne et plus préférentiellement encore un extrait de bois de châtaignier.

D'une manière avantageuse, on utilise l'extrait végétal dans une quantité de 0,01 à 1 %, de préférence de 0,05 à 0,3 % et plus préférentiellement encore d'environ 0,15 % en poids par rapport au poids d'aliments solides ou fibreux donnés à l'animal.

L'extrait végétal peut être obtenu par les méthodes connues de l'homme de l'art telles qu'une extraction alcoolique ou une extraction à l'eau. On préfère ici les extraits végétaux aqueux.

En effet, lorsqu'on donne au moins un tanin hydrolysable (ellagitanin) tel que défini ci-dessus à un animal, notamment un mammifère et plus particulièrement un veau, on constate un abaissement du taux de fer dans le sang et donc une diminution de l'hématocrite tout en respectant les valeurs individuelles et moyennes d'un lot de veaux tel que défini par la réglementation européenne.

Le tanin hydrolysable peut être incorporé dans la ration alimentaire ou donné séparément. La ration alimentaire est généralement composée d'au moins un aliment fibreux et d'aliments liquides (aliments d'allaitement, eau).

Ainsi, le tanin hydrolysable peut être utilisé de trois manières :
1) En « sécurité » : avec des aliments fibreux dosant 40 à 50 ppm maximum de fer, la couleur de la viande peut être dégradée. Ainsi le tanin hydrolysable permet de sécuriser cette éventualité.
2) En « correctif » : avec des aliments fibreux dosant 40 à 50 ppm maximum de fer, le tanin hydrolisable peut être apporté en « post feeding », c'est-à-dire en fin de période d'élevage lorsque le niveau d'hématocrite est trop élevée. L'emploi du tanin hydrolisable permet de maîtriser ce dernier et ainsi rétablir une couleur de viande conforme.
3) En « alimentaire » : dans ce cas, le tanin hydrolisable est donné aux animaux en dose adaptée par rapport aux aliments fibreux dont la teneur en fer peut dépasser 40 à 50 ppm ce qui permet ainsi un plus grand choix de nutriments possibles sans risquer de dégrader la couleur de la viande.

Dans les différents cas d'utilisation, le tanin peut être ajouté aux aliments par plusieurs voies, notamment dans la ration alimentaire, c'est-à-dire dans l'aliment solide, et/ou dans l'aliment liquide (par exemple aliment d'allaitement, eau), ou séparément, ou par une combinaison quelconque de ces voies.

Dans un mode de réalisation particulier, le tanin hydrolysable est incorporé dans la ration alimentaire de l'animal. Dans une variante, cette ration alimentaire comprend un aliment fibreux contenant de préférence plus de 40 ppm, plus préférentiellement plus de 50 ppm de fer.

D'excellents résultats ont été obtenus avec un extrait aqueux de bois de châtaignier.

L'invention concerne également un aliment pour animaux ainsi qu'un complément alimentaire pour animaux comprenant au moins un tanin hydrolysable tel que défini ci-dessus.

Du fait que les tanins hydrolysables (ellagitanins) tels que définis dans la présente demande permettent de réduire l'absorption du fer alimentaire au niveau de l'appareil digestif, les tanins hydrolysables tels que définis dans la présente demande sont également utiles dans le traitement de maladies caractérisées par une absorption excessive du fer alimentaire (hémochromatose). L'invention concerne donc également un tanin hydrolysable pour utilisation comme médicament pour réduire le taux d'absorption de fer alimentaire par un patient, notamment pour le traitement de l'hémochromatose.

L'invention est décrite plus en détail ci-après, à l'aide de l'exemple suivant qui est nullement limitatif mais est donné à titre d'exemple uniquement.

### EXEMPLE - Elevage de veaux de boucherie

Deux formules d'aliment d'allaitement couramment employées ont été comparées.

### CONDITIONS EXPERIMENTALES

### 1. - Durée d'élevage : 24 semaines

### 2. - Animaux et schéma expérimental

40 veaux mâles de race Prime Holstein âgés de 8 à 10 jours et d'un poids vif de 45 à 50 kg sont répartis en deux lots de 20 répartis dans des cases de 5 veaux en se référant à leur poids vif et à leur hématocrite à J29 (29^{ième} jour de la période expérimentale).

Jour 29 à Jour 168 de la période expérimentale: régimes expérimentaux :

### Lot Témoin

### Lot Expérimental avec de l'extrait acqueux de bois de châtaignier (0,15 %)

Les formules des aliments de la période expérimentale (Jour 29 à Jour 168) et leurs caractéristiques principales sont indiquées dans les tableaux 1 et 1 bis.

L'extrait aqueux de bois de châtaignier est commercialisé par la société ECOPSI (Arras, France).

**Tableau 1: Composition des aliments d'allaitement utilisés dans les régimes expérimentaux**

| | **Croissance (J29 -J85)** | | **Finition (J86 - J168)** | |
|---|---|---|---|---|
| **Matières premières (% en poids)** | **Expérimental** | **Témoin** | **Expérimental** | **Témoin** |
| **Lacto doux** | 22.77 | 20.74 | 27.08 | 31.92 |
| **Lacto R40 suif** | *18.50* | 18.3 | 27.07 | 21.62 |
| **Lacto R50 saindoux** | 11.97 | 12.8 | 8.85 | 9.82 |
| **Lacto R40 coprah** | 11.57 | 11.44 | 9.85 | 9.82 |
| **WPC (contentré de protéines sériques)** | 20.23 | 21.2 | 3.25 | 2.82 |
| **Hydrolysat de protéines de poisson** | 5.0 | 0 | 5.0 | 0 |
| **Extrait aqueux de bois de châtaignier** | 0.15 | 0 | 0.15 | 0 |
| **Farine de blé** | 0 | 0 | 0 | 3 |
| **Farine de blé prégel** | 0 | 0 | 5 | 5.00 |
| **Lactose** | 0 | 0 | 8.25 | 7.64 |
| **Farine de pois extrudée** | 3.0 | 0 | 3 | 3 |
| **Soja** | 4.0 | 4.0 | 0.80 | 2.5 |
| **Prémélange (vitamines, minéraux, oligo-éléments)** | 1.0 | 1.0 | 1.0 | 1.0 |
| **Carbonate de Ca** | 0.6 | 0.6 | 0.65 | 0.72 |
| **Gluten** | 4.97 | 8.54 | 1.97 | 5.36 |
| **Phosphate** | 0.27 | 0.2 | 0.18 | 0.50 |
| **Formiate de Ca** | 0.30 | 0.3 | 0.3 | 0.3 |
| **Tixosil™** | 0.22 | 0.22 | 0.22 | 0.52 |
| **Lysine** | 0.30 | 0.5 | 0.33 | 0.50 |
| **Thréonine** | 0 | 0.04 | 0.07 | 0.14 |
| **A135** | 0.13 | 0.12 | 0.13 | 0.12 |

**Tableau 1 bis: Valeurs nutritionnelles des aliments d'allaitement utilisés**

| *Aliments* | | *Croissance* | | *Finition* | |
|---|---|---|---|---|---|
| | | *Témoin* | *Expérimental* | *Témoin* | *Expérimental* |
| **Valeurs calculées** | | | | | |
| Matières azotées digestibles | % | 21.78 | 21.71 | 13.97 | 14.2 |
| Energie digestible | kcal/kg | 4624 | 4621 | 4540 | 4528 |
| Matières grasses totales | % | 19.33 | 19.33 | 20.00 | 19.90 |
| | | 17.41 | 17.44 | 11.68 | |
| **Lysine digestible** | g/kg | | | | 12.14 |
| M+C digestible | g/kg | 9.39 | 9.39 | 6.16 | 6.32 |
| Thréonine digestible | g/kg | 11.88 | 11.95 | 8.14 | 8.10 |
| Tryptophane digestible | g/kg | 2.89 | 2.82 | 1.58 | 1.59 |
| Leucine digestible | g/kg | 16.49 | 13.58 | 8.86 | 5.82 |
| Isoleucine digestible | g/kg | 9.09 | 7.51 | 4.93 | 3.27 |
| Valine digestible | g/kg | 9.18 | 7.48 | 5.0 | 3.22 |
| Histidine digestible | g/kg | 3.73 | 2.92 | 2.14 | 1.3 |
| Ca | g/kg | 7.29 | 7.29 | 7.01 | 9.1 |
| P | g/kg | 5.25 | 5.26 | 5.28 | 7.7 |
| NaCl | g/kg | 14.39 | 21.73 | 18.9 | 19.6 |
| K | g/kg | 17 | 14.24 | 13.65 | 15.7 |
| Fer disponible (estimation) | ppm | 15 | 16.5 | 6.9 | 7 |

Les veaux sont alimentés au seau. Les aliments d'allaitement sont distribués sous forme de deux buvées quotidiennes selon les plans précisés en annexes 1 et 1 bis, soit 14 repas par semaine du début jusqu'à la fin de l'essai.

### 4. Alimentation fibreuse

La distribution d'aliments fibreux (bouchons à base de céréales) s'effectue environ 1 heure après la buvée du matin, après vérification de la propreté des seaux. La quantité apportée par veau est de 50 g à 100 g/jour de la 2^{ème} semaine à la 14^{ème} semaine incluse, au-delà, la distribution passe à 250 g/j jusqu'à l'abattage.

**Tableau 2 : Composition chimique du bouchon à base de céréales et de légumineuses**

| | | |
|---|---|---|
| *Protéines brutes* | : | *13.7 %* |
| *Matières grasses brutes* | : | *2,5 %* |
| *Cellulose brute* | : | *9.6 %* |
| *Cendres* | : | *3.7 %* |
| *Fer (ppm)* | : | *<50* |

### 5. - Mesures : hématocrites toutes les 4 semaines

### Evolution de l'hématocrite

En cours d'élevage, les hématocrites se différencient significativement. Il est à remarquer que l'hématocrite est plus faible dans le lot expérimental, malgré les recharges en fer réalisées. Les veaux du lot expérimental ont en effet reçu, à chaque période, des recharges de fer dextran plus marquées que les veaux des autres lots.

**Tableau 3 : Evolution de l'hématocrite (%)**

| *Régime* | *Témoin* | *Expérimental* |
|---|---|---|
| ***J29*** | *29,1 ± 4,5* | *29,1 ± 4,5* |
| ***J56*** | *28,5 ± 2,7* | *26,1 ± 2,9* |
| ***J85*** | *25,8 ± 1,7* | *24,2 ± 2,0* |
| ***J113*** | *27,4 ± 2,2* | *23,6 ± 1,7* |
| ***J141*** | *26,2 ± 3,1* | *22,1 ± 2,0* |
| ***J167*** | *24,8 ± 1,7* | *22,1 ± 2,7* |

La maîtrise de l'hématocrite des animaux est appréciée par le pourcentage d'éléments figurés dans le sang, lui-même lié au nombre et à la taille des globules rouges présents. L'hématocrite moyen des veaux à la mise en lots est proche de 29,1.

En cours d'élevage, les apports de fer disponible dans l'aliment d'allaitement sont normalement limités afin d'obtenir un taux d'hématocrite adapté et consécutivement une viande claire à l'abattoir. Un hématocrite voisin de 23 à 24% est actuellement considéré comme un objectif.

La mesure régulière des hématocrites individuels permet de contrôler et de comparer l'évolution de l'hématocrite selon les différents lots. Selon la valeur de l'hématocrite, des recharges sous forme de fer dextran sont réalisées.

En cours d'élevage, les hématocrites se différencient significativement. Les veaux du lot expérimental ont reçu, à chaque période, des recharges de fer dextran plus marquées que les veaux du lot témoin.

Néanmoins, l'hématocrite des veaux du lot expérimental est resté inférieur. Cette baisse de l'hématocrite dans le lot expérimental est attribuée à la présence d'ellagitanins, apportés par l'ajout à l'aliment d'extrait aqueux de bois de châtaignier, qui chélatent le fer et le rendent ainsi non absorbable au niveau de l'appareil digestif.

L'exemple montre donc clairement que les ellagitanins, font baisser le taux de fer dans le sang des veaux.

### Evolution des poids vifs et des croissances (tableaux 4 et 5)

**Tableau 4 : Evolution des poids vifs (kg)**

| *Régime* | *Témoin* | *Expérimental* |
|---|---|---|
| ***J29*** | *62,3 ± 2,5* | *62.3 ± 2,9* |
| ***J56*** | *90,9 ± 4,2* | *91.5 ± 2,7* |
| ***J85*** | *125,5 ± 5,3* | *129,1 ± 4,8* |
| ***J113*** | *157,8 ± 8,4* | *159,5 ± 10,4* |
| ***J141*** | *193,9 ± 12,5* | *193,7 ± 14,4* |
| ***J167*** | *226,1 ± 21,4* | *224,6 ± 21,5* |

Les poids vifs des deux lots ne se différencient ni par leur performance moyenne, ni par leur hétérogénéité.

**Tableau 5: Evolution des GMQ (g/j)**

| *Régime* | *Témoin* | *Expérimental* |
|---|---|---|
| ***Croissance (1-85j)*** | *1129 ± 81* | *1192 ± 88* |
| ***Finition (86-168j)*** | *1227 ± 227* | *1165 ± 237* |
| ***Totalité (1-168j)*** | *1187 ± 154* | *1175 ± 151* |

Le calcul des croissances journalières confirme les observations liées à l'évolution des poids vifs. En période de croissance le lot témoin serait désavantagé (P<0,012) par rapport au lot expérimental. Globalement les croissances enregistrées dans les lots se hiérarchisent conformément aux poids vifs observés.

Dans le lot témoin, les croissances sont plus faibles que celles habituellement observées avec un régime sans poudre de lait écrémé. Les croissances enregistrées sont généralement situées entre de 1250g et 1300g sur la totalité de la période d'élevage excluant la phase de démarrage de J1 à 128.

### 6. - Indices de consommation (tableau 6)

**Tableau 6 : Indices de consommation**

| *Régime* | *Témoin* | *Expérimental* |
|---|---|---|
| ***Indice réel global*** | *1,99 ± 0,21* | *1,98 ± 0,19* |

Sur la période globale, l'indice de consommation des deux lots est quasi identique.

### 7. - Caractéristiques des carcasses (tableau7)

**Tableau 7: Principaux résultats d'engraissement et caractéristiques des carcasses**

| *Régime* | *Témoin* | *Expérimental* |
|---|---|---|
| *Poids vif début essai (kg)* | *62,3 ± 2,5* | *62,3 ± 2,9* |
| *Poids vif fin (kg)* | *226,1 ± 27,4* | *224,6 ± 21,5* |
| *Poids de carcasse froide (kg)* | *126,95 ± 13,4* | *125.6 ± 13,2* |
| *Rendement en carcasse (%)* | *56,2 ± 0,01* | *55,8 ± 0,01* |
| *Caractéristiques des carcasses:* | | |
| *- couleur (1 à 4)¹* | *2,45 ± 0,5* | *2,25 ± 0,44* |
| *- conformation (1* à *18)²* | *8,0 ± 1,2* | *7,95 ± 0,68* |
| *- état d'engraissement (1 à 4)³* | *2,85 ± 0,4* | *2,85 ± 0,49* |

| | | |
|---|---|---|
| ¹1=*«blanc»* à 4 = *«rouge»* ²1 = *« très peu de muscle »* à 18 = *« musculature hyper développée »* ³1 = *« très maigre* » à 4 = « *très gras* » | | |

Conformément aux poids vifs, les poids de carcasse des deux lots ne se différencient pas. Les rendements sont similaires. Il est de même pour l'engraissement et la conformation. Toutefois, la couleur du lot expérimental a tendance à être plus claire comme le laissait présager le niveau d'hématocrite.

Le présent exemple montre donc clairement que le fait d'ajouter les ellagitanins, dans l'alimentation permet d'obtenir une viande très claire sans diminution des performances zootechniques.

## Revendications

1. Utilisation non-thérapeutique d'au moins un ellagitanin pour réduire l'absorption par un animal du fer ingéré, notamment contenu dans un aliment.

2. Utilisation selon la revendication 1, **caractérisée en ce que** l'animal est un mammifère et notamment un veau.

3. Utilisation selon la revendication 1 ou 2, **caractérisée en ce que** l'ellagitanin est incorporé à la ration alimentaire de l'animal.

4. Utilisation selon la revendication 3, **caractérisé en ce que** la ration alimentaire comprend un aliment fibreux contenant plus de 40 ppm, de préférence plus de 50 ppm de fer.

5. Utilisation selon l'une quelconque des revendications 1 à 4, **caractérisée en ce que** l'ellagitanin est utilisé sous forme d'un extrait végétal.

6. Utilisation selon la revendication 5 **caractérisée en ce que** l'extrait végétal est un extrait de bois de châtaignier.

7. Utilisation selon la revendication 6, **caractérisé en ce que** l'extrait de bois de châtaignier est employé à une dose de 0.01 à 1% par rapport au poids d'aliments solides donnés à l'animal.

8. Aliment pour animaux comprenant un extrait végétal tel que défini dans l'une quelconque des revendications 5 à 7.

9. Complément alimentaire pour animaux comprenant un extrait végétal tel que défini dans l'une quelconque des revendications 5 à 7.

10. Ellagitanin pour utilisation comme médicament pour réduire le taux d'absorption de fer alimentaire par un patient.
